# EUROPÄISCHE PATENTSCHRIFT

(11) **EP 4 108 175 B1**
(45) Veröffentlichungstag und Bekanntmachung des Hinweises auf die Patenterteilung: **28.02.2024**
(21) Anmeldenummer: 22174650.6
(22) Anmeldetag: 20.05.2022
(51) Int. Cl.: A61B 6/03, A61B 6/00, H04L 1/24

(54) **VERFAHREN ZUR ÜBERWACHUNG EINER DATENÜBERTRAGUNG, VORRICHTUNG, INSBESONDERE ZUR MEDIZINISCHEN BILDGEBUNG, UND COMPUTERPROGRAMM**
COMPUTER PROGRAM AND METHOD FOR MONITORING A DATA TRANSMISSION, DEVICE, IN PARTICULAR FOR MEDICAL IMAGING
PROCÉDÉ DE SURVEILLANCE D'UNE TRANSMISSION DE DONNÉES, DISPOSITIF, EN PARTICULIER D'IMAGERIE MÉDICALE ET PROGRAMME INFORMATIQUE

(30) Priorität: 23.06.2021 DE 102021206494
(43) Veröffentlichungstag der Anmeldung: 28.12.2022
(73) Patentinhaber: Siemens Healthineers AG, 91301 Forchheim (DE)
(72) Erfinder: Greif, Peter, 91361 Pinzberg/Gosberg (DE); Karl, Harald, 90765 Fürth (DE); Kiesel, Jutta, 91301 Forchheim (DE); Urban, Andreas, 90542 Eckental (DE); Welker, Ludwig, 91330 Eggolsheim (DE)
(74) Vertreter: Siemens Healthineers Patent Attorneys

(56) Entgegenhaltungen:
- DE-A1-102005 027 632
- DE-A1-102005 035 207
- DE-B3- 10 322 138

## Beschreibung

Die Erfindung betrifft ein Verfahren zur Überwachung einer Datenübertragung zwischen einer ersten und zweiten Komponente, wobei durch eine Änderung einer Position und/oder einer Orientierung der ersten Komponente bezüglich der zweiten Komponente eine oder mehrere ortsfest an der ersten Komponente angebrachte Sendeeinrichtung oder Sendeeinrichtungen jeweils bezüglich wenigstens zweier ortsfest an der zweiten Komponente angebrachter Empfangseinrichtungen bewegt werden. Daneben betrifft die Erfindung eine Vorrichtung, die insbesondere zur medizinischen Bildgebung dient, ein Computerprogramm und einen computerlesbaren Datenträger.

Im Bereich der medizinischen Bildgebung, beispielsweise bei Computertomographen, aber auch in anderen Anwendungsgebieten kann es erforderlich sein, Daten zwischen relativ zueinander bewegten Komponenten zu übertragen. So sind beispielsweise die bildgebenden Röntgensensoren bei Computertomographen typischerweise an einer bezüglich einer Basis drehbar gelagerten Gantry angeordnet und die Datenverarbeitung bzw. Visualisierung soll durch bezüglich der Basis feststehende Komponenten, beispielsweise Arbeitsplatzrechner oder feststehende Bildprozessoren, erfolgen.

Prinzipiell kann eine solche Datenübertragung beispielsweise über Schleifkontakte erfolgen. Um hohe Datenraten bei geringerem Wartungsaufwand und geringerer Störanfälligkeit zu erreichen, kann es jedoch vorteilhaft sein, stattdessen eine drahtlose Datenübertragung über kurze Strecken zu nutzen. Die Datenübertragung kann hierbei beispielsweise per Funk bzw. allgemein durch Hochfrequenztechnik oder über eine kapazitive Kopplung erfolgen.

Durch Nutzung einer kurzreichweitigen Drahtloskommunikation kann eine hohe Datenrate und eine geringe Störanfälligkeit erreicht werden. Auch in der Umgebung befindliche weitere Elektronik wird durch eine kurzreichweitige Kommunikation nicht oder wenig gestört. Es ist es jedoch möglich, dass aufgrund der Relativbewegung von Sender und Empfänger sich vorhandene Empfänger vorübergehend, beispielsweise innerhalb eines gewissen Drehwinkelbereichs einer Gantry, in einem Bereich befinden, in dem von keinem der Sender eine ausreichende Signalstärke empfangen werden kann, um eine robuste Kommunikation zu ermöglichen.

So beschreibt beispielsweise die DE102005035207 A1 ein Verfahren und eine Vorrichtung zur Datenübertragung zwischen zwei relativ zueinander bewegten Komponenten eines Computertomographen.

Während im regulären Betrieb ein Datenverlust beispielsweise durch ein redundantes Empfangen von Datenpaketen über mehrere an unterschiedlichen Positionen der Gantry angeordnete Empfänger vermieden werden kann, erschwert die beschriebene Problematik eine Diagnose der die Datenübertragung nutzenden Vorrichtung erheblich. Würden feste Paare aus Sender und Empfänger zur drahtlosen Kommunikation genutzt, könnte eine Störung dieser Kommunikation bzw. ein Ausfall des Senders oder des Empfängers sehr einfach detektiert werden, indem beispielsweise eine empfängerseitig erfasste Signalstärke, Datenrate oder Anzahl von erfolgreich empfangenen Paketen überwacht wird. Beispielsweise könnte, wenn eine der genannten Größen unter einen vorgegebenen Grenzwert fällt, ein Fehlerzähler erhöht werden und bei Erreichen eines vorgegebenen Wertes durch den Fehlerzähler kann beispielsweise ein Hinweis an einen Nutzer bzw. Servicepersonal gegeben werden. Würde ein solches Vorgehen jedoch im obig beschriebenen Verfahren genutzt, so würden typischerweise auch bei einem tatsächlich störungsfreien Betrieb eine hohe Zählrate eines solchen Fehlerzählers auftreten, da er stets erhöht würde, wenn eine der Empfangseinrichtungen aus den Senderbereichen der Sendeeinrichtungen herausbewegt wird.

Der Erfindung liegt somit die Aufgabe zugrunde, ein demgegenüber verbessertes Verfahren zur Überwachung einer Datenübertragung anzugeben, dass insbesondere auch bei einer Nutzung von relativ zueinander bewegten Komponenten, zwischen denen Daten übertragen werden sollen, eine robuste Fehlerdetektion ermöglicht.

Die Aufgabe wird erfindungsgemäß durch ein Verfahren der eingangs genannten Art gelöst, wobei bei Erfüllung einer Fehlerbedingung für die jeweilige Empfangseinrichtung ein jeweiliger Fehler registriert wird und/oder der Betrieb einer die erste und zweite Komponente umfassenden Vorrichtung modifiziert wird, wobei die Erfüllung der Fehlerbedingung für die jeweilige Empfangseinrichtung sowohl von einer ermittelten, die Position und/oder Orientierung der ersten Komponente bezüglich der zweiten Komponente betreffenden Lageinformation als auch von einem Maß für eine Empfangsqualität der von der Sendeeinrichtung oder von wenigstens einer der Sendeeinrichtungen empfangenen Signale und/oder Datenpakete abhängt.

Durch die zusätzliche Berücksichtigung der Lageinformation im Rahmen der Fehlerbedingung ist es im erfindungsgemäßen Verfahren möglich, im Rahmen der Fehlererkennung nur solche Betriebssituationen zu berücksichtigen, in denen aufgrund der relativen Position der Sendeeinrichtungen zu der jeweiligen Empfangseinrichtung bei einem störungsfreien Betrieb überhaupt eine hinreichende Empfangsqualität an der Empfangseinrichtung zu erwarten ist. Somit können insbesondere nur Zeiträume bei der Auswertung der Fehlerbedingung berücksichtigt werden, zu denen bei einem störungsfreien Betrieb und funktionstüchtigen Sende- und Empfangseinrichtungen eine hinreichend hohe Empfangsqualität erwartet wird. Betriebssituationen, in denen aufgrund der Relativposition bzw. -orientierung der Komponenten systembedingt eine geringere Empfangsqualität erwartet wird, können unberücksichtigt bleiben. Somit weist in dem erfindungsgemäßen Verfahren die Erfüllung der Fehlerbedingung auf das tatsächliche Vorliegen einer Störung oder eines Defekts hin und die eingangs diskutierte Problematik wird vermieden.

Die Übertragung von der Sendeeinrichtung zur Empfangseinrichtung kann insbesondere drahtlos erfolgen. Wie obig erläutert, kann in diesem Fall durch die relative Bewegung der Komponenten beispielsweise eine Empfangsantenne der Empfangseinrichtung aus einem Abstrahlbereich der jeweiligen Sendeeinrichtung herausbewegt werden, in dem eine ausreichende Signalqualität zu erwarten ist. Alternativ wäre beispielsweise eine drahtgebundene Übertragung, z.B. über einen Schleifkontakt, möglich, wobei der Schleifkontakt beispielsweise nur in bestimmten Positionen bzw. Orientierungen geschlossen ist. Beispielsweise kann bei einer drehbaren Lagerung der ersten Komponente an der zweiten Komponente oder umgekehrt ein solcher Schleifkontakt nur über einen Teil des Rotationswinkels in Umfangsrichtung geführt sein.

Unter dem Registrieren eines Fehlers kann beispielsweise das Hochzählen eines Fehlerzählers, insbesondere eines der jeweiligen Empfangseinrichtung zugeordneten Fehlerzählers, verstanden werden. Alternativ oder ergänzend ist es auch möglich, dass ein Fehler registriert wird, indem ein entsprechender Eintrag, insbesondere mit einem zugeordnetem Zeitstempel, in ein Log-File geschrieben wird oder Ähnliches.

Die Modifikation des Betriebs der Vorrichtung kann dazu dienen, einen Hinweis an einen lokalen Nutzer oder, beispielsweise über das Internet oder ein Mobilfunkprotokoll, an einen beabstandeten Nutzer bzw. einen Server zu geben. Es ist jedoch auch möglich, unmittelbar oder z.B. bei Erreichen einer gewissen Fehlerzahl bzw. -häufigkeit den Betrieb der Vorrichtung zumindest teilweise einzustellen, also beispielsweise eine Rotation einer Komponente zu unterbinden, eine Röntgenröhre abzuschalten oder Ähnliches. Dies kann zweckmäßig sein, da starke Störungen der Datenübertragung potenziell zu einem Datenverlust führen können, womit es, beispielsweise im Bereich der medizinischen Bildgebung, zweckmäßig sein kann, eine Untersuchung abzubrechen und somit z.B. einen unnötigen Eintrag von Röntgenstrahlung in ein Untersuchungsobjekt zu vermeiden.

Über die jeweilige Sendeeinrichtung kann eine Sequenz von mehreren aufeinanderfolgenden Datenpaketen gesendet werden, wobei das jeweilige Datenpaket eine Sequenzinformation umfasst, die die Position des jeweiligen Datenpakets in der Sequenz beschreibt, wobei nach Empfang mehrerer Datenpakete durch jeweils wenigstens eine der Empfangseinrichtungen eine Paketverlustbedingung ausgewertet wird, deren Erfüllung von den Sequenzinformationen der empfangenen Datenpakete abhängt und indiziert, dass wenigstens eines der Datenpakete der Datensequenz durch keine der Empfangseinrichtungen empfangen wurde, wobei einerseits die Fehlerbedingung nur bei Erfüllung der Paketverlustbedingung erfüllbar ist und/oder wobei andererseits bei Erfüllung der Paketverlustbedingung ein Paketverlust registriert wird und/oder der Betrieb der die erste und zweite Komponente umfassenden Vorrichtung modifiziert wird.

Durch die zusätzliche Auswertung der Paketverlustbedingung wird einerseits eine erheblich robustere Erkennung von Fehlern bzw. Störungen bei der Datenübertragung ermöglicht und andererseits werden zusätzliche Diagnoseinformationen gewonnen, die es beispielsweise ermöglichen können, Defekte zu lokalisieren.

Werden z.B. einfache Fehlerzähler genutzt, die jedoch, wie obig erläutert, durch Berücksichtigung der Lageinformation beispielsweise in bestimmten relativen Orientierungen der Komponenten zueinander nicht erhöht werden, tritt hierbei potentiell das Problem auf, dass beispielsweise aufgrund einer eingeschränkten Granularität der Lageinformation aus Implementierungsgründen die Bereiche, in denen keine Fehlererkennung erfolgen soll, nur relativ grob definiert werden können. In diesem Fall müsste entweder in Kauf genommen werden, dass auch bei einem nicht gestörten Betrieb bereits hohe Fehlerzählraten auftreten, was wie obig erläutert eine Fehlererkennung erheblich erschwert, oder es müsste in Kauf genommen werden, dass bestimmte tatsächlich auftretende Fehler maskiert werden. Zudem kann durch isolierte Fehlerzähler für einzelne Empfangseinrichtungen nicht erkannt werden, ob beispielsweise ein Datenpaket, während dessen Empfang an einer bestimmten Empfangseinrichtung ein Fehler aufgetreten ist, über eine andere Empfangseinrichtung korrekt empfangen wurde.

Beide Nachteile können durch zusätzliche Auswertung einer Paketverlustbedingung vermieden werden. Beispielsweise können durch eine jeweilige Sendeeinrichtung gesendete Datenpakete sequentiell nummeriert werden. Es kann somit leicht erkannt werden, wenn Lücken in der Sequenz auftreten. Die Sequenzinformation kann beispielsweise durch die Sendeeinrichtung selbst, z. B. als fortlaufende Nummer, zum Datenpaket hinzugefügt werden oder sie kann bereits vor der Übermittlung an die Sendeeinrichtung, beispielsweise durch einen Router, der zu übertragende Pakete auf verschiedene Sendeeinrichtungen verteilt, hinzugeführt werden.

Wie später noch genauer erläutert werden wird, können im Rahmen der Auswertung der Paketverlustbedingung insbesondere empfange Pakete von allen Empfangseinrichtungen aggregiert werden. Während dies eine robuste Erkennung ermöglicht, ob ein Datenpaket tatsächlich durch eine der Empfangseinrichtungen empfangen wurde, ist jedoch im Falle eines Paketverlusts nicht oder zumindest nicht ohne erheblichen Zusatzaufwand erkennbar, wo im System überhaupt eine Störung vorlag. Daher ist es besonders vorteilhaft, die Auswertung der Paketverlustbedingung und die obig erläuterte kombinierte Auswertung des Maßes für die Empfangsqualität und der Lageinformation zu kombinieren, um Störungen, die tatsächlich zu Paketverlusten führen, konkret einer bestimmten Empfangseinrichtung zuordnen zu können oder beispielsweise um einen an einer Empfangseinrichtung detektierten Fehler nur dann als kritisch zu betrachten und somit zu zählen, wenn er auch tatsächlich zu einem verlorenen Paket führt.

Hierbei ist es möglich, die Fehlerbedingung und die Paketverlustbedingung zunächst separat zu prüfen und Fehler und Paketverluste, beispielsweise durch separate Zähler, separate Log-Files oder auch in einem gemeinsamen Log-File zu erfassen. Die Fehlerdiagnose für eine im Rahmen der Datenübertragung genutzte Vorrichtung bzw. eine die Datenübertragung implementierende Vorrichtung kann dann durch Auswertung entsprechender Zähler oder Dateien in einem nachgelagerten Vorgang erfolgen. Es kann jedoch vorteilhaft sein, die beiden Kriterien gemeinsam zu überwachen und somit eine Fehlerbedingung zu nutzen, die zusätzlich die Paketverlustbedingung auswertet.

Das erfindungsgemäße Verfahren, insbesondere die Auswertung der Fehler- und/oder Paketverlustbedingung, kann beispielsweise durch eine entsprechend programmierte Datenverarbeitungseinrichtung implementiert sein, also insbesondere ein computerimplementiertes Verfahren sein. Alternativ können die entsprechenden Verarbeitungsschritte beispielsweise durch eine feste Verdrahtung bzw. einen ASIC implementiert sein.

Die Datenpakete können zusätzlich eine Senderinformation umfassen, die beschreibt, von welcher der Sendeeinrichtungen das jeweilige Datenpaket gesendet wurde, wobei die Paketverlustbedingung jeweils separat für mehrere Gruppen von Datenpaketen, deren Senderinformationen dieselbe Sendeeinrichtung beschreiben, ausgewertet wird. Die Senderinformation kann durch die Sendeeinrichtungen selbst oder beispielsweise durch einen Router, der die Datenpakete auf die verschiedenen Sendeeinrichtungen verteilt, zum jeweiligen Datenpaket hinzugefügt werden. Durch Auswertung der Senderinformation kann beispielsweise leicht identifiziert werden, wenn die Paketverlustbedingung erfüllt ist, weil eine bestimmte Sendeeinrichtung defekt oder besonders störanfällig ist. Eine solche Information ist im Rahmen der Diagnose hochrelevant, da sie beispielsweise auf eine erforderliche Wartung hinweisen kann bzw. eine Wartung deutlich erleichtern kann, da bereits bekannt ist, welche Komponente defekt bzw. besonders störanfällig ist.

Zudem erleichtert es die zusätzliche Berücksichtigung der Senderinformation erheblich, zu erkennen, ob alle Datenpakete einer Sequenz empfangen wurden. Werden mehrere Sendeeinrichtungen genutzt, können diese nämlich gleichzeitig oder zumindest überlappend verschiedene Datenpakete senden, sodass unter Umständen keine eindeutige Paketreihenfolge festgelegt werden kann. Werden die durch die verschiedenen Sendeeinrichtungen übertragenen Datenpakte jedoch separat für die einzelnen Sendeeinrichtungen betrachtet, resultiert eine klare Reihenfolge und somit eine einfache Identifikation der einzelnen Datenpakete, beispielsweise durch eine Sequenznummer.

Von einer jeweiligen Sendeeinrichtung empfangene Datenpakete oder zumindest die Sequenzinformation der empfangenden Datenpakte betreffende Teilinformationen der Datenpakete können in einem Pufferspeicher abgelegt werden, wobei jeweils bei oder nach Erfüllung einer ersten Prüfbedingung, deren Erfüllung von einer Anzahl und/oder einer Gesamtgröße der in einem Teilpuffer des Puffers abgelegten Datenpakete oder Teilinformation abhängt, oder bei oder nach Erfüllung einer zweiten Prüfbedingung, deren Erfüllung einerseits von einer Anzahl und/oder einer Gesamtgröße der nach Erfüllung der ersten Prüfbedingung in einem weiteren Teilpuffer des Puffers abgelegten Datenpakete oder Teilinformationen abhängt und/oder andererseits von einer seit Erfüllung der ersten Prüfbedingung verstrichenen Zeit abhängt, zur Prüfung der Paketverlustbedingung die in dem ersten Teilpuffer abgelegten Datenpakete oder Teilinformationen oder die in dem ersten und zweiten Teilpuffer abgelegten Datenpakete oder Teilinformationen ausgewertet werden.

Durch ein Zwischenspeichern der Datenpakete bzw. Teilinformationen in einem Pufferspeicher kann bereits mit geringerem Aufwand geprüft werden, ob die dort gespeicherten Sequenzinformationen auf Lücken hinweisen, beispielsweise weil bei einer fortlaufenden Paketnummerierung der Datenpakete bestimmte Paketnummern fehlen.

Der Ansatz, dass bereits bei hinreichender Befüllung eines Teilpuffers, das heißt bei Erfüllung der ersten Prüfbedingung, unmittelbar die Paketverlustbedingung auf diesem Teilpuffer ausgewertet wird, stößt jedoch an Grenzen, wenn aufgrund unterschiedlicher Übertragungswege, insbesondere aufgrund des Empfangs durch verschiedene Empfangseinrichtungen, Datenpakete in einer anderen Reihenfolge empfangen werden können, als sie gesendet wurden. In diesem Fall kann es vorteilhaft sein, nach Erfüllung der ersten Prüfbedingung eintreffende Datenpakete, die im ersten Teilpuffer noch vorhandene Lücken potenziell füllen können, zusätzlich zu berücksichtigen und somit, wie obig erläutert, eine zweite Prüfbedingung zu nutzen und erst bei oder nach Erfüllung von dieser zweiten Prüfbedingung Datenpakete bzw. Teilinformationen aus beiden Teilpuffern im Rahmen der Paketverlustbedingung zu berücksichtigen.

Eine relativ einfache Möglichkeit, das beschriebene Vorgehen umzusetzen, ist es, einen Wechselpuffer zu nutzen, sodass empfangene Datenpakete, insbesondere empfangene Datenpakete, die von einer bestimmten Sendeeinrichtung stammen, unabhängig davon, über welche der Empfangseinrichtungen sie empfangen wurden, zunächst in den ersten Teilpuffer geschrieben werden, wobei bei einem gewissen Füllgrad bzw. einer gewissen Anzahl von Einträgen in diesem Puffer ein Wechsel zum zweiten Teilpuffer erfolgt, in den nach Erfüllung der ersten Prüfbedingung empfange Datenpakete geschrieben werden. Etwas später, also bei Erfüllung der zweiten Prüfbedingung, können dann die im ersten Teilpuffer gespeicherten Daten gemeinsam mit den darüberhinausgehend im zweiten Teilpuffer gespeicherten Daten gemeinsam ausgewertet werden. Das Schreiben zusätzlicher Datenpakete muss hierzu nicht unterbrochen werden und die erste Prüfungsbedingung kann beispielsweise erneut erfüllt sein, wenn der zweite Teilpuffer hinreichend gefüllt ist, wonach wiederum ein Wechsel zum ersten Teilpuffer erfolgt, der die weiteren Datenpakete speichert, bis die zweite Prüfbedingung erfüllt ist. Statt der gesamten Datenpakete können natürlich auch nur Teildaten der jeweiligen Datenpakete im jeweiligen Teilpuffer gespeichert werden, wie bereits obig erläutert wurde.

Die erste Komponente kann drehbar an der zweiten Komponente gelagert sein oder umgekehrt, wobei der Drehwinkel zwischen der ersten und zweiten Komponente oder eine mit diesem Drehwinkel korrelierte Information als Lageinformation verwendet wird. Insbesondere kann somit die relative Position und Orientierung der ersten und zweiten Komponente nach dem Drehwinkel von 360° der Position und Orientierung bei einem Drehwinkel von 0° entsprechen. Der Drehwinkel kann beispielsweise über einen Drehwinkelsensor, beispielsweise ein Hall-Sensor, erfasst werden oder beispielsweise aufgrund einer entsprechenden Ansteuerung eines Aktors, der die Komponenten relativ zueinander bewegt, bekannt sein.

Die Fehlerbedingung kann ausschließlich dann erfüllbar sein, wenn der Drehwinkel in einem vorgegebenen Drehwinkelbereich oder in einem von mehreren vorgegebenen Drehwinkelbereichen liegt.

Allgemein kann die Fehlerbedingung nur dann erfüllbar sein, wenn eine die Lageinformation auswertende Teilbedingung erfüllt ist. Die Teilbedingung bzw. die Vorgabe wenigstens eines Drehwinkelbereichs kann somit als eine Art Filter für die Erfüllung der Fehlerbedingung dienen und beispielsweise kann das Hochzählen eines Fehlerzählers unterdrückt werden, wenn der Drehwinkel in keinem der vorgegebenen Drehwinkelbereiche liegt bzw. die Teilbedingung nicht erfüllt ist.

Der vorgegebene Drehwinkelbereich bzw. die vorgegebenen Drehwinkelbereiche können insbesondere für zumindest ein Paar von Empfangseinrichtungen voneinander unterschiedlich sein, das heißt, dass für bestimmte Drehwinkel die Erfüllung der Fehlerbedingung nur für Teile der Empfangseinrichtungen bzw. nur für eine der Empfangseinrichtungen unmöglich ist. Unabhängig davon können für jede Empfangseinrichtung insbesondere genauso viele Drehwinkelbereiche definiert sein, wie Sendeeinrichtungen vorhanden sind. Insbesondere werden genau jene Drehwinkelbereiche vorgegeben, in denen erwartungsgemäß von wenigstens einer Sendeeinrichtung ein Signal mit hinreichender Qualität empfangbar sein sollte.

Die Anzahl der verwendeten Empfangseinrichtungen kann um genau eins oder wenigstens zwei größer sein als die Zahl der verwendeten Sendeeinrichtungen. Die Sende- und Empfangseinrichtungen könnten so an der ersten und zweiten Komponente verteilt werden, beispielsweise entlang der Umfangsrichtung einer drehbar gelagerten Komponente, dass ein von einer Sendeeinrichtung abgestrahltes Signal bzw. Datenpaket im Regelbetrieb ohne Störungen von wenigstens einer der Empfangseinrichtungen empfangen werden kann. Ein Empfang eines gesendeten Datenpakets durch mehrere der Empfangseinrichtungen ist typischerweise unproblematisch, da mehrfach empfangene Datenpakete, beispielsweise auf Basis der Sequenzinformation oder aufgrund von anderen im Datenpaket enthaltenden Informationen, erkannt werden können und somit Doubletten verworfen werden können.

Neben dem erfindungsgemäßen Verfahren betrifft die Erfindung eine Vorrichtung mit einer ersten und zweiten Komponente, wobei durch eine Änderung einer Position und/oder einer Orientierung der ersten Komponente bezüglich der zweiten Komponente eine oder mehrere ortsfest an der ersten Komponente angebrachte Sendeeinrichtung oder Sendeeinrichtungen jeweils bezüglich wenigstens zweier ortsfest an der zweiten Komponente angebrachter Empfangseinrichtungen bewegbar sind, wobei die Vorrichtung Mittel umfasst, die geeignet sind, das erfindungsgemäße Verfahren auszuführen. Insbesondere umfasst die Vorrichtung als Mittel oder als Teil der Mittel einer Datenverarbeitungseinrichtung, die dazu eingerichtet bzw. programmiert ist, die Fehlerbedingung auszuwerten. Ergänzend oder alternativ kann die Datenverarbeitungseinrichtung dazu eingerichtet bzw. programmiert sein, die Paketverlustbedingung auszuwerten.

Die Lageinformation kann sensorisch, beispielsweise über einen Drehwinkelsensor, erfasst werden, jedoch auch dadurch bekannt sein, dass die Datenverarbeitungseinrichtung oder eine Steuereinrichtung der Vorrichtung wenigstens einen Aktor steuert, der die Bewegung bzw. Orientierungsänderung zwischen den Komponenten vorgibt. Ausgehend von einer definierten oder erfassten Ausgangsstellung kann dann auf Basis von Ansteuerinformationen die momentane Stellung ermittelt werden.

Die Mittel können mehrteilig sein und beispielsweise eine jeweilige Datenverarbeitungseinrichtung für die erste und zweite Komponente umfassen. Die Datenverarbeitungseinrichtung der ersten Komponente kann dazu eingerichtet bzw. programmiert sein, eine Aufteilung von zu sendenden Daten auf Datenpakete vorzunehmen, diese Datenpakete auf die Sendeeinrichtungen zu verteilen und/oder Informationen zu den Datenpaketen, insbesondere Sequenzinformation bzw. Senderinformation, hinzuzufügen. Eine derart eingerichtete bzw. programmierte Datenverarbeitungseinrichtung kann den obig diskutierten Router bilden. Die Datenverarbeitungseinrichtung der zweiten Komponente kann insbesondere zur Prüfung der Fehler- bzw. Paketverlustbedingung dienen.

Die zweite Komponente kann eine feststehende Basis sein, an der die erste Komponente beweglich, insbesondere drehbar, gelagert ist oder umgekehrt. Der Bewegungspfad der Sendeeinrichtungen bezüglich der zweiten Komponente kann insbesondere zyklisch sein, sich also nach einer bestimmten Bewegungsstrecke bzw. einem bestimmten Drehwinkel wiederholen. Diese ist beispielsweise bei einer drehbaren Lagerung der zweiten Komponente an der ersten Komponente oder umgekehrt der Fall.

Die Vorrichtung kann zur medizinischen Bildgebung dienen und insbesondere ein Computertomograph sein. Ergänzend oder alternativ kann die erste Komponente wenigstens einen bildgebenden Sensor tragen, beispielsweise einen Röntgendetektor. Bilddaten des bildgebenden Sensors können als Datenpakete über die Sendeeinrichtungen übertragen werden. Insbesondere kann zwischen den Sensor und die Sendeeinrichtungen ein Router geschaltet sein, der die Daten auf die verschiedenen Sendeeinrichtungen verteilt. Hierbei kann es ausreichend sein, jedes Datenpaket genau einer Sendeeinrichtung zum Senden zuzuführen, um zu erreichen, dass bei einem ordnungsgemäßen Betrieb trotz der Relativbewegung der Komponenten kein Datenpaket verloren geht.

Die Erfindung betrifft zudem ein Computerprogramm, umfassend Befehle, die bewirken, dass die erfindungsgemäße Vorrichtung das erfindungsgemäße Verfahren ausführt. Insbesondere dient das Computerprogramm dazu, dass Mittel der Vorrichtung, also insbesondere wenigstens eine Datenverarbeitungseinrichtung, zu programmieren.

Die Erfindung betrifft zudem einen computerlesbaren Datenträger, auf dem ein erfindungsgemäßes Computerprogramm gespeichert ist.

Weitere Vorteile und Einzelheiten der Erfindung ergeben sich aus den folgenden Ausführungsbeispielen und den zugehörigen Figuren. Hierbei zeigen schematisch:
- Fig. 1: ein Ausführungsbeispiel einer erfindungsgemäßen Vorrichtung, die im Beispiel zur medizinischen Bildgebung dient, und die zur Durchführung eines Ausführungsbeispiels des erfindungsgemäßen Verfahrens zur Überwachung einer Datenübertragung eingerichtet ist,
- Fig. 2: eine Darstellung der zur Überwachung der Datenübertragung in Fig. 1 relevanten Elemente,
- Fig. 3: Diagramme zur Auswertung einer Fehlerbedingung in dem Ausführungsbeispiel des erfindungsgemäßen Verfahrens, und
- Fig. 4: ein Ablaufdiagramm des Ausführungsbeispiels des erfindungsgemäßen Verfahrens.

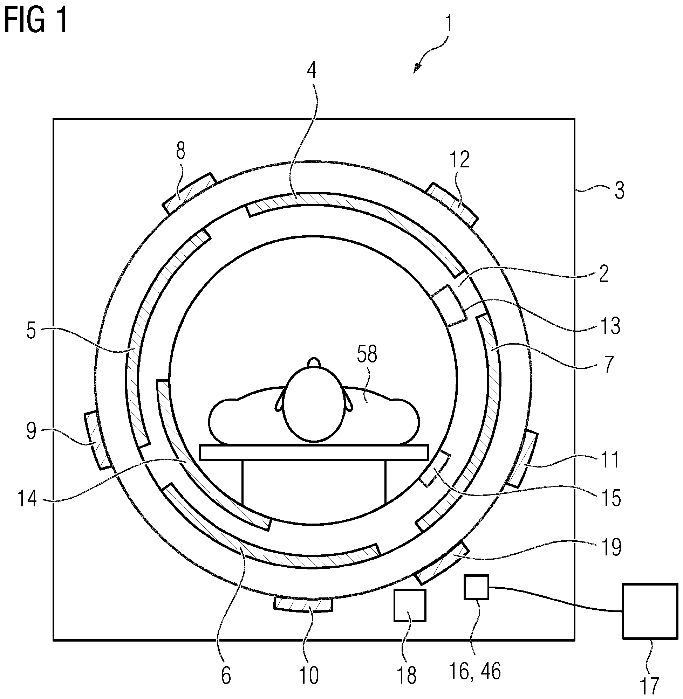

Fig. 1 zeigt eine Vorrichtung 1 mit einer ersten und zweiten Komponente 2, 3, die relativ zueinander beweglich, im Beispiel drehbar, sind, und zwischen denen eine Datenübertragung erfolgen soll. Im Beispiel dient die Vorrichtung zur medizinischen Bildgebung, wobei beispielhaft ein Computertomograph dargestellt ist. In diesem Beispiel werden somit Bilddaten des Untersuchungsobjekts 58 erfasst, indem über eine Röntgenröhre 13 Röntgenstrahlung abgestrahlt wird, die nach Durchstrahlung des Untersuchungsobjekts 58 durch den bildgebenden Sensor 14, der im Beispiel ein Röntgendetektor ist, erfasst wird.

Die resultierten Bilddaten sollen an eine externe Einrichtung 17 bereitgestellt werden. Da jedoch die Komponente 2, die den bildgebenden Sensor 14 trägt, beweglich, im Beispiel drehbar, bezüglich der Komponente 3, die eine Datenverarbeitungseinrichtung 16 trägt, die die Daten an die externe Einrichtung 17 bereitstellen soll, gelagert ist, ist eine Datenübertragung zwischen beweglich zueinander gelagerten Komponenten erforderlich. Diese wird im Beispiel dadurch implementiert, dass die Bilddaten zunächst durch einen Router 15 in mehrere Datenpakete aufgeteilt werden, wobei für jedes der Datenpakete eine von mehreren vorhandenen Sendeeinrichtungen 4, 5, 6, 7 gewählt wird, über die dieses Datenpaket drahtlos abgestrahlt wird.

Dadurch, dass mehr Empfangseinrichtungen 8-12 vorhanden sind als Sendeeinrichtungen und eine geeignete Verteilung der Sende- und Empfangseinrichtungen 4-12 gewählt wird, kann erreicht werden, dass stets wenigstens eine der Empfangseinrichtungen 8-12 von einer jeweiligen Sendeeinrichtung 4-7 abgestrahlte Signale bzw. Datenpakete empfangen kann. Die durch die verschiedenen Empfangseinrichtungen 8-12 empfangenen Datenpakete werden durch die Datenverarbeitungseinrichtung 16 zusammengeführt, wobei insbesondere mehrfach empfangene Datenpakete aussortiert werden können, und an die externe Einrichtung 17 weitergeleitet.

Insbesondere im Bereich der medizinischen Bildgebung soll zügig erkannt werden, wenn, beispielsweise aufgrund einer externen Störung oder eines Defekts einer der Sende- bzw. Empfangseinrichtungen 4-12 die Datenübertragung gestört ist, was unter Umständen zu einem Paketverlust und somit beispielsweise zu unbrauchbaren Messungen führen kann.

Würden die Sendeeinrichtungen 4-7 jeweils fest einer der Empfangseinrichtungen 8-12 zugeordnet sein, könnte eine Störung bzw. ein Defekt eines bestimmten Kanals, das heißt eines bestimmten Paars von Sende- und Empfangseinrichtungen 4-12 relativ einfach dadurch erkannt werden, dass ein Maß für eine Empfangsqualität bestimmt wird, beispielsweise eine Signalamplitude oder eine Paketverlustrate der einzelnen Empfangseinrichtung 8-12, und bei zu schlechter Empfangsqualität, also beispielsweise wenn das Maß einen Grenzwert unterschreitet, ein Fehler registriert wird bzw. aufgrund dieses Fehlers der Betrieb der Vorrichtung 1 modifiziert wird, beispielsweise die Röntgenröhre 13 deaktiviert wird oder Ähnliches.

Da aufgrund der Relativbewegung von Sende- und Empfangseinrichtungen 4-12 einzelne Empfangseinrichtungen 8-12 jedoch vorübergehend von keiner der Sendeeinrichtung 4-7 ein Signal empfangen bzw. nur ein Signal mit sehr schlechter Qualität empfangen, würde ein derartiges Vorgehen selbst bei einem tatsächlich störungsfreien Betrieb bereits Fehler registrieren.

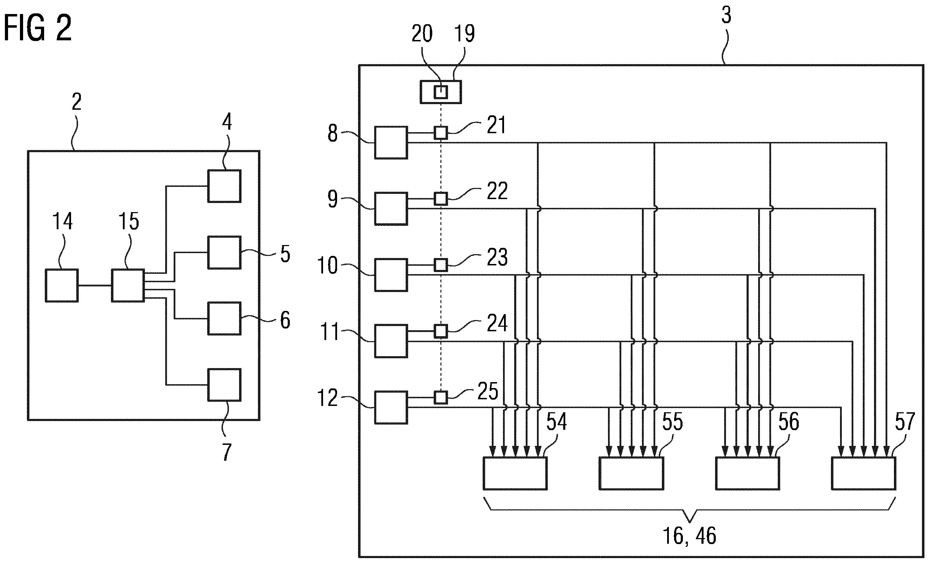

Daher wird ein anderes Verfahren zur Fehlererkennung genutzt, dass im Folgenden mit zusätzlichem Bezug auf Fig. 2 näher erläutert wird. Die für die Komponente 2 dargestellten relevanten Elemente entsprechen hierbei, abgesehen von ihrer deutlich vereinfachten Darstellung, den bereits mit Bezug auf Fig. 1 dargestellten Elementen. Für die Komponente 3 ist jedoch die Weiterverarbeitung der durch die Empfangseinrichtungen 8-12 empfangenen Datenpakete durch mehrere Funktionsblöcke dargestellt, die durch Mittel 46 der Vorrichtung, im Beispiel ebenfalls die Datenverarbeitungseinrichtung 16, implementiert werden.

Hierbei wird zunächst für jede der Empfangseinrichtungen 8-12 separat eine Fehlerbedingung 21-25 ausgewertet. Die jeweilige Fehlerbedingung 21-25 hängt einerseits von einem Maß für eine Empfangsqualität der durch die jeweilige Empfangseinrichtung 8-12 von den Sendeeinrichtungen 4-7 empfangenen Signale bzw. Datenpakete ab. Um jedoch die obig beschriebene Problematik zu vermeiden, dass bei ausschließlicher Berücksichtigung eines solchen Maßes auch bei fehlerfreiem Funktionieren der Vorrichtung 1 eine solche Fehlerbedingung häufig erfüllt wäre, wertet die jeweilige Fehlerbedingung 21-25 zusätzlich eine Lageinformation 20 aus, die die Position bzw. im Beispiel die Orientierung der ersten Komponente 2 bezüglich der zweiten Komponente 3 betrifft. Im Beispiel wird ein relativer Drehwinkel der ersten Komponente 2 bezüglich der zweiten Komponente 3 durch einen Drehwinkelsensor 19 erfasst. Alternativ oder ergänzend wäre es beispielsweise auch möglich, einen solchen Drehwinkel aus Steuersignalen der Datenverarbeitungseinrichtung 16 zu Ansteuerung eines Aktors 18 zur Drehung der Komponente 2 zu bestimmen oder Ähnliches.

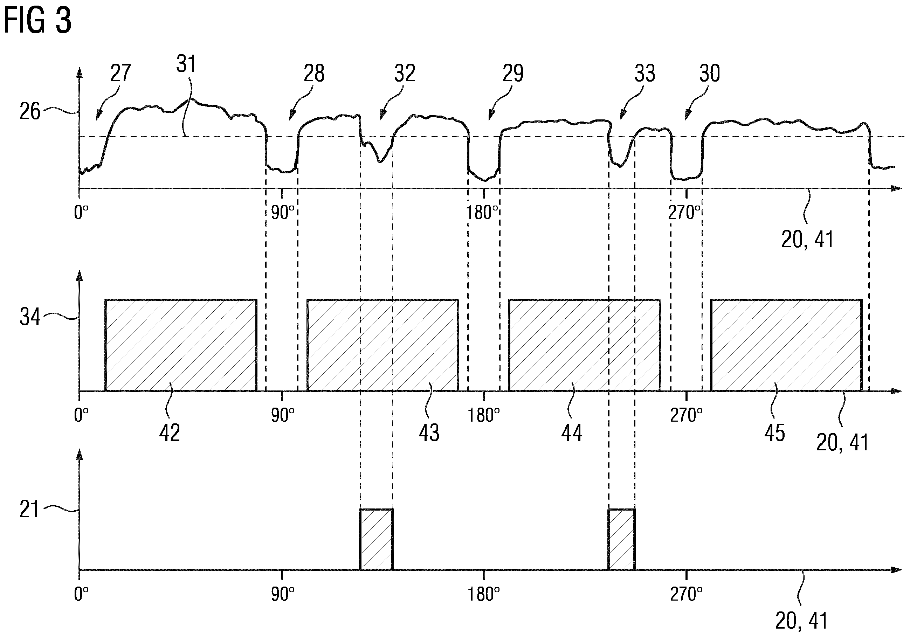

Ein Beispiel für die Auswertung der Fehlerbedingungen 21-25 wird im Folgenden mit zusätzlichem Bezug auf Fig. 3 für die Fehlerbedingung 21 näher erläutert. Hierbei zeigt Fig. 3 drei Diagramme mit gleicher X-Achse, auf der die Lageinformation 20, also im Beispiel der Drehwinkel 41 der Komponente 2 bezüglich der Komponente 3, aufgetragen ist.

Im obersten Diagramm stellt die Y-Achse das Maß 26 für die Empfangsqualität dar, also beispielsweise eine Signalstärke des empfangenen Signals oder ein Signal-zu-Rausch-Verhältnis. Die dargestellte Kurve zeigt einen beispielhaften Verlauf dieses Maßes 26 für eine Umdrehung der Komponente 2. Hierbei ist es zu erkennen, dass das Maß 26 für die Empfangsqualität der Empfangseinrichtung 8 jeweils deutlich abfällt, wenn sich die Empfangseinrichtung 8 aufgrund der Rotation der Komponente 2 in einem Bereich zwischen zwei Sendeeinrichtungen 4-7 in Umfangsrichtung der Komponente 2 betrachtet befindet. Die Reduzierung des Maßes 26 für die Empfangsqualität in den Zeitintervallen 27, 28, 29 und 30 ist somit systemimmanent und soll nicht als Fehler erkannt werden.

Im Beispiel sind zusätzliche Abfälle des Maßes 26 für die Empfangsqualität in den Zeitintervallen 32 und 33 eingezeichnet, die sich nicht aus der Systemgeometrie ergeben und beispielsweise durch externe Störungen oder Defekte an der Empfangseinrichtung 8 bzw. an den Sendeeinrichtung 8-12 verursacht sein könnten. In diesen Zeitintervallen 32, 33 soll somit ein Fehler erkannt werden, um eine Diagnose zu ermöglichen.

Um dies zu erreichen, wird eine Fehlerbedingung 21 genutzt, die nur dann überhaupt erfüllbar ist, wenn eine Teilbedingung 34 erfüllt wird, die, wie schematisch im mittleren Diagramm der Fig. 3 dargestellt ist, genau dann erfüllt ist, wenn der Drehwinkel 41 in einem der markierten Drehwinkelbereiche 42, 43, 44 oder 45 liegt.

Als weitere Teilbedingung, die erfüllt sein muss, um die Fehlerbedingung zu erfüllen, wird eine Unterschreitung des Grenzwerts 31 durch das Maß 26 für die Empfangsqualität gefordert. Dies führt, wie schematisch im untersten Diagramm in Fig. 3 dargestellt ist, dazu, dass die Fehlerbedingung 21 ausschließlich in den Zeitintervallen 32 und 33 erfüllt ist und nicht in den Zeitintervallen 27, 28, 29 und 30, also zum gewünschten Verhalten.

Prinzipiell wäre die Auswertung der Fehlerbedingungen 21-25 zu Diagnose- bzw. Steuerzwecken bereits ausreichend. Hierbei wäre jedoch problematisch, dass je nach Wahl der Ausdehnung der Drehwinkelbereiche 42-45 entweder bestimmte Fehler nicht erkannt werden oder weiterhin unter Umständen Fehler erkannt werden, wenn tatsächlich kein Fehler vorliegt. Zudem kann bei Nutzung relativ einfacher Ansätze zur Auswertung der Erfüllung der Fehlerbedingung, also beispielsweise dann, wenn bei jeder Erfüllung der Fehlerbedingung ein Fehlerzähler erhöht wird, nicht ohne weiteres erkannt werden, ob ein Fehler beispielsweise durch eine bestimmte der Sendeeinrichtungen 4-7 erzeugt wurde.

Zur weiteren Verbesserung der Fehlerüberwachung werden somit in dem in Fig. 2 gezeigten Beispiel zusätzlich die Paketverlustbedingungen 54-57 ausgewertet. Hierbei prüft die Paketverlustbedingung 54 einen Verlust von Paketen der Sendeeinrichtung 4, die Paketverlustbedingung 55 einen Verlust von Paketen der Sendeeinrichtung 5, die Paketverlustbedingung 56 den Verlust von Paketen der Sendeeinrichtung 6 und die Paketverlustbedingung 57 den Verlust von Paketen der Sendeeinrichtung 7.

Somit kann eindeutig festgestellt werden, ob ein Paketverlust und somit ein tatsächlicher Fehler vorliegt oder nicht. Da zudem die Fehlerbedingungen 21-25 ausgewertet werden, kann bei empfangsseitiger Verursachung eines Fehlers im Gegensatz zu einer ausschließlichen Auswertung von Paketverlusten ohne weiteres eindeutig erkannt werden, für welche der Empfangseinrichtungen 8-12 Fehler auftreten.

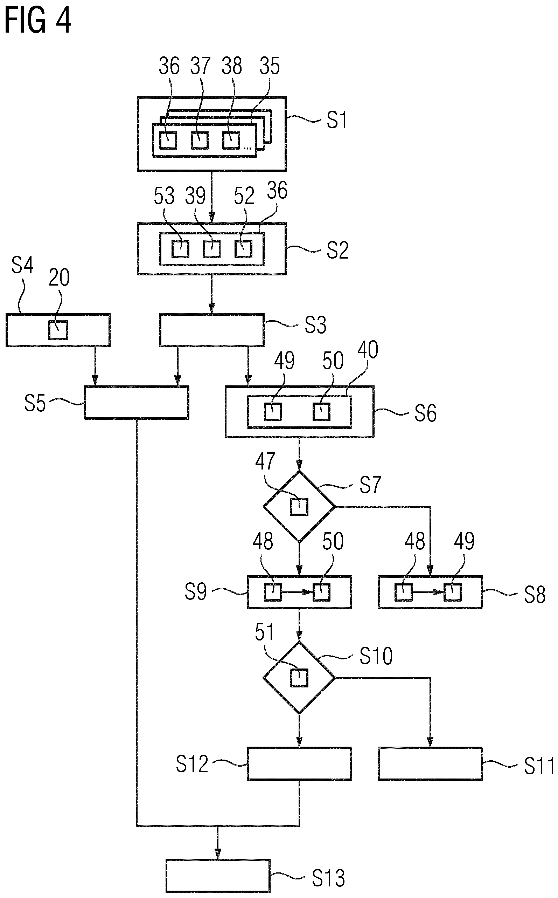

Eine konkrete Implementierung der Auswertung der Paketverlustbedingung 54-57 im Rahmen eines Verfahrens zur Überwachung einer Datenübertragung wird im Folgenden mit Bezug auf das in Fig. 4 schematisch dargestellte Ablaufdiagramm eines solchen Verfahrens erläutert.

In Schritt S1 werden die durch den bildgebenden Sensor 14 bereitgestellten Bilddaten durch den Router 15 in mehrere Sequenzen von aufeinanderfolgenden Datenpaketen 36-38 aufgeteilt, wobei für jede der Sendeeinrichtungen 4-7 eine separate Sequenz 35 bereitgestellt wird. Insbesondere kann hierbei jedes zu übertragende Datenpaket nur in genau einer der Sequenzen 35 enthalten sein.

In Schritt S2 wird das jeweilige Datenpaket, wie schematisch für das Datenpaket 36 dargestellt ist, durch Zusatzinformationen erweitert. Dies kann durch den Router 15 oder durch die jeweilige zum Senden genutzte Sendeeinrichtung 4-7 erfolgen. Neben den im Beispiel durch den Sensor 14 bereitgestellten Nutzdaten 52 umfasst das Datenpaket 36-38 eine Sequenzinformation 39, die die Position des jeweiligen Datenpakets in der Sequenz 35 beschreibt. Beispielsweise kann ein Zähler für jedes Datenpaket um 1 erhöht werden und als Sequenzinformation 39 in dem jeweiligen Datenpaket 36-38 gespeichert werden. Zusätzlich wird in dem jeweiligen Datenpaket 36-38 eine Senderinformation 53 ergänzt, die beschreibt, von welcher der Sendeeinrichtungen 4-7 das jeweilige Datenpaket gesendet wird.

Im Schritt S3 werden die entsprechend ergänzten Datenpakete 36-38 der jeweiligen Sequenz 35 durch die jeweilige Sendeeinrichtung 4-7 gesendet und, soweit keine Störungen auftreten, durch jeweils wenigstens eine der Empfangseinrichtungen 8-12 empfangen. Gleichzeitig mit dem Senden eines jeweiligen Datenpakets 36-38 durch eine jeweilige Sendeeinrichtung 4-7 wird in Schritt S4 die Lageinformation 20 erfasst, um anschließend in Schritt S5, wie bereits obig erläutert, die jeweilige Fehlerbedingung 21-25 für die jeweilige Empfangseinrichtung 8-12 auszuwerten.

In Schritt S6 werden die durch die verschiedenen Empfangseinrichtungen 8-12 empfangenen Datenpakete 36-38 aggregiert und gemäß der in ihnen gespeicherten Senderinformation 53 in separate Gruppen sortiert, wobei die folgenden Schritte nur beispielhaft für eine diese Gruppen erläutert werden sollen.

Die Datenpakete 36-38 einer Gruppe bzw. zumindest eine jeweilige Teilinformation 48 jedes Datenpakets 36-38 der Gruppe, die zumindest die Sequenzinformation 39 umfasst, sollen bzw. soll in einem Puffer 40 abgelegt werden, der durch die beiden Teilpuffer 49 und 50 gebildet wird, wobei die Teilpuffer 49 und 50 insbesondere als Wechselpuffer genutzt werden können.

Hierzu wird in Schritt S7 zunächst eine erste Prüfbedingung 47 geprüft, deren Füllung von der Anzahl oder Gesamtgröße der im Teilpuffer 49 bereits abgelegten Datenpaket bzw. Teilinformationen 48 abhängt. Insbesondere kann diese erste Prüfbedingung erfüllt sein, wenn der erste Teilpuffer 49 vollständig gefüllt ist. Ist dies nicht der Fall, so kann in Schritt S8 die Teilinformation 48 bzw. das Datenpaket 36-38 in den Teilpuffer 49 geschrieben werden, womit die Auswertung der jeweiligen Paketverlustbedingung 54-57 zunächst, solange bis ein weiteres Datenpaket der entsprechenden Gruppe empfangen wird, abgeschlossen ist.

Ist die erste Prüfbedingung 47 hingegen erfüllt, so wird in Schritt S9 die Teilinformation 48 stattdessen in den Teilpuffer 50 geschrieben und anschließend in Schritt S10 eine zweite Prüfbedingung 51 ausgewertet, deren Erfüllung von der Anzahl bzw. Gesamtgröße der nach Erfüllung der ersten Prüfbedingung 47 in den Teilpuffer 50 abgelegten Datenpakete abhängt. Alternativ oder ergänzend kann eine Erfüllung der zweiten Prüfbedingung auch von einer seit Erfüllung der ersten Prüfbedingung 47 verstrichenen Zeit abhängen.

Ist die zweite Prüfbedingung 51 nicht erfüllt, so wird die Auswertung der jeweiligen Paketverlustbedingung 54-57 in Schritt S11 solange unterbrochen, bis ein weiteres Datenpaket 36-38 der jeweiligen Gruppe empfangen wird. Anders ausgedrückt dient die Auswertung der zweiten Prüfbedingung 51 dazu, nach Erfüllung der ersten Prüfbedingung 47 für eine gewisse Zeit oder eine gewisse Anzahl bzw. eine gewisse Größe von empfangenen Paketen zu warten und erst bei Erfüllung der zweiten Prüfbedingung 51 in Schritt S12 die im Puffer 40 bzw. in den Teilpuffern 49,50 abgelegten Datenpakete 36-38 bzw. Teilinformation 48 zu prüfen, um einen Paketverlust zu erkennen.

Da die gespeicherten Datenpakete 36-38 bzw. Teilinformationen 48 die Sequenzinformation 39 umfassen, kann beispielsweise geprüft werden, ob für jedes Datenpaket 36-38, das bzw. dessen Teilinformation 48 im Teilpuffer 49 gespeichert ist, ein Datenpaket 36-38 bzw. eine Teilinformation 48 mit der unmittelbar folgenden Sequenzinformation 39 in dem Puffer 40, also im Teilpuffer 49 oder im Teilpuffer 50, gespeichert ist. Ist dies der Fall, so ist zumindest während des Zeitintervalls, während dem in den Teilpuffer 49 geschrieben wurde, kein Paketverlust aufgetreten. Andererseits kann, wenn dies nicht der Fall ist, von einem Paketverlust ausgegangen werden, zumindest dann, wenn sichergestellt ist, dass ausreichend viele empfangene Pakete bzw. ausreichend Zeit zwischen der Erfüllung der ersten und zweiten Prüfbedingung 47, 51 liegen.

Somit kann in Schritt S12 entschieden werden, ob die jeweilige Paketverlustbedingung 54-57 für die jeweilige Sequenz 35 von Datenpaketen 36-38 bzw. die jeweilige Sendeeinrichtung 4-7 erfüllt ist oder nicht. Zudem kann für spätere Durchläufe die Rolle der Teilpuffer 49 und 50 getauscht werden, sodass zunächst bei Nichterfüllung der ersten Prüfbedingung weiterhin in den Teilpuffer 50 geschrieben wird und erst hiernach ein Rückwechsel auf den Teilpuffer 49 erfolgt.

In Schritt S13 wird das Ergebnis der Auswertung der Fehlerbedingungen 22-25 in Schritt S5 und das Ergebnis der Auswertung der Paketverlustbedingungen 54-57 in Schritt S12 gemeinsam weiterverarbeitet. Alternativ wäre es z.B. auch möglich, entsprechende Daten zunächst separat, beispielsweise in separaten Log-Files bzw. über separate Fehlerzähler, auszuwerten und eine zusammenfassende Analyse erst nachgelagert durchzuführen.

Die gemeinsame Verarbeitung in Schritt S13 kann beispielsweise dazu dienen, ein aussagekräftiges Log-File bereitzustellen, dass bereits Informationen enthalten kann, ob gezählte Fehler bzw. ein erkannter Paketverlust durch bestimmte Sendeeinrichtungen 4-7 oder bestimmte Empfangseinrichtungen 8-12 erzeugt wurden oder ob eine solche Zuordnung nicht möglich ist.

Eine gemeinsame Verarbeitung ist besonders zweckmäßig, wenn in den Betrieb der Vorrichtung 1 eingegriffen werden soll, da beispielsweise bei Erkennen eines einzelnen Paketverlusts es ausreichend sein kann, dieses Paket erneut zu senden, während beispielsweise ein gemeinsames Auftreten von einzelnen Paketverlusten mit einem hohen Wert eines Fehlerzählers, der bei Erfüllung einer bestimmten der Fehlerbedingungen 21-25 erhöht wird, durchaus auf eine defekte Empfangseinrichtung 8-12 hinweisen kann, weshalb in diesem Fall beispielsweise ein weiterer Betrieb der Vorrichtung 1 unterbunden werden kann, bis eine Wartung erfolgt ist.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Verfahren zur Überwachung einer Datenübertragung zwischen einer ersten und zweiten Komponente (2, 3), wobei durch eine Änderung einer Position und/oder einer Orientierung der ersten Komponente (2) bezüglich der zweiten Komponente (2) eine oder mehrere ortsfest an der ersten Komponente (2) angebrachte Sendeeinrichtung (4 - 7) oder Sendeeinrichtungen (4 - 7) jeweils bezüglich wenigstens zweier ortsfest an der zweiten Komponente (3) angebrachter Empfangseinrichtungen (8 - 12) bewegt werden, **dadurch gekennzeichnet, dass** bei Erfüllung einer Fehlerbedingung (21 - 25) für die jeweilige Empfangseinrichtung (8 - 12) ein jeweiliger Fehler registriert wird und/oder der Betrieb einer die erste und zweite Komponente (2, 3) umfassenden Vorrichtung (1) modifiziert wird, wobei die Erfüllung der Fehlerbedingung (21 - 25) für die jeweilige Empfangseinrichtung (8 - 12) sowohl von einer ermittelten, die Position und/oder Orientierung der ersten Komponente (2) bezüglich der zweiten Komponente (3) betreffenden Lageinformation (20) als auch von einem Maß (26) für eine Empfangsqualität der von der Sendeeinrichtung (4 - 7) oder von wenigstens einer der Sendeeinrichtungen (4 - 7) empfangenen Signale und/oder Datenpakete (36 - 38) abhängt.

2. Verfahren nach Anspruch 1, **dadurch gekennzeichnet, dass** über die jeweilige Sendeeinrichtung (4 - 7) eine Sequenz (35) von mehreren aufeinanderfolgenden Datenpaketen (36 - 38) gesendet wird, wobei das jeweilige Datenpaket (36 - 38) eine Sequenzinformation (39) umfasst, die die Position des jeweiligen Datenpakets (36 - 38) in der Sequenz (35) beschreibt, wobei nach Empfang mehrerer Datenpakete (36 - 38) durch jeweils wenigstens eine der Empfangseinrichtungen (8 - 12) eine Paketverlustbedingung (54 - 57) ausgewertet wird, deren Erfüllung von den Sequenzinformationen (39) der empfangenen Datenpakete (36 - 38) abhängt und indiziert, dass wenigstens eines der Datenpakete (36 - 38) der Sequenz (35) durch keine der Empfangseinrichtungen (8 - 12) empfangen wurde, wobei einerseits die Fehlerbedingung (21 - 25) nur bei Erfüllung der Paketverlustbedingung (54 - 57) erfüllbar ist und/oder wobei andererseits bei Erfüllung der Paketverlustbedingung (54 - 57) ein Paketverlust registriert wird und/oder der Betrieb der die erste und zweite Komponente (2, 3) umfassenden Vorrichtung (1) modifiziert wird.

3. Verfahren nach Anspruch 2, **dadurch gekennzeichnet, dass** die Datenpakete (36 - 38) zusätzlich eine Senderinformation (53) umfassen, die beschreibt, von welcher der Sendeeinrichtungen (4 - 7) das jeweilige Datenpaket (36 - 38) gesendet wurde, wobei die Paketverlustbedingung (54 - 57) jeweils separat für mehrere Gruppen von Datenpaketen (36 - 38), deren Senderinformationen (53) dieselbe Sendeeinrichtung (4 - 7) beschreiben, ausgewertet wird.

4. Verfahren nach Anspruch 2 oder 3, **dadurch gekennzeichnet, dass** von einer jeweiligen Sendeeinrichtung (4 - 7) empfangene Datenpakete (36 - 38) oder zumindest die Sequenzinformation (53) der empfangenen Datenpakete (36 - 38) betreffende Teilinformationen (48) der Datenpakete (36 - 38) in einem Pufferspeicher (40) abgelegt werden, wobei jeweils bei oder nach Erfüllung einer ersten Prüfbedingung (47), deren Erfüllung von einer Anzahl und/oder einer Gesamtgröße der in einem Teilpuffer (49) des Puffers (40) abgelegten Datenpakete (36 - 38) oder Teilinformationen (48) abhängt,
oder bei oder nach Erfüllung einer zweiten Prüfbedingung (51), deren Erfüllung einerseits von einer Anzahl und/oder einer Gesamtgröße der nach Erfüllung der ersten Prüfbedingung (47) in einem weiteren Teilpuffer (50) des Puffers (40) abgelegten Datenpakete (36 - 38) oder Teilinformationen (48) abhängt und/oder andererseits von einer seit Erfüllung der ersten Prüfbedingung (47) verstrichenen Zeit abhängt,
zur Prüfung der Paketverlustbedingung (54 - 57) die in dem ersten Teilpuffer (49) abgelegten Datenpakete (36 - 38) oder Teilinformationen (48) oder die in dem ersten und zweiten Teilpuffer (49, 50) abgelegten Datenpakete (36 - 38) oder Teilinformationen (48) ausgewertet werden.

5. Verfahren nach einem der vorangehenden Ansprüche,
**dadurch gekennzeichnet, dass** die erste Komponente (2) drehbar an der zweiten Komponente (3) gelagert ist oder umgekehrt, wobei der Drehwinkel (41) zwischen der ersten und zweiten Komponente (2, 3) oder eine mit diesem Drehwinkel (41) korrelierte Information als Lageinformation (20) verwendet wird.

6. Verfahren nach Anspruch 5, **dadurch gekennzeichnet, dass** die Fehlerbedingung (21 - 25) ausschließlich dann erfüllbar ist, wenn der Drehwinkel (41) in einem vorgegebenen Drehwinkelbereich (42 - 45) oder in einem von mehreren vorgegebenen Drehwinkelbereichen (42 - 45) liegt.

7. Verfahren nach einem der vorangehenden Ansprüche,
**dadurch gekennzeichnet, dass** die Fehlerbedingung (21 - 25) nur dann erfüllbar ist, wenn eine die Lageinformation (20) auswertende Teilbedingung (34) erfüllt ist.

8. Verfahren nach einem der vorangehenden Ansprüche,
**dadurch gekennzeichnet, dass** die Anzahl der verwendeten Empfangseinrichtungen (8 - 12) um genau eins oder um wenigstens zwei größer ist als die Zahl der verwendeten Sendeeinrichtungen (4 - 7).

9. Vorrichtung mit einer ersten und zweiten Komponente (2, 3), wobei durch eine Änderung einer Position und/oder einer Orientierung der ersten Komponente (2) bezüglich der zweiten Komponente (3) eine oder mehrere ortsfest an der ersten Komponente (2) angebrachte Sendeeinrichtung (4 - 7) oder Sendeeinrichtungen (4 - 7) jeweils bezüglich wenigstens zweier ortsfest an der zweiten Komponente (3) angebrachter Empfangseinrichtungen (8 - 12) bewegbar sind, **dadurch gekennzeichnet, dass** die Vorrichtung Mittel (46) umfasst, die geeignet sind, das Verfahren nach einem der vorangehenden Ansprüche auszuführen.

10. Vorrichtung nach Anspruch 9, **dadurch gekennzeichnet, dass** die Vorrichtung (1) zur medizinischen Bildgebung dient und/oder dass die erste Komponente (2) wenigstens einen bildgebenden Sensor (14) trägt.

11. Computerprogramm, umfassend Befehle, die bewirken, dass die Vorrichtung (1) des Anspruchs 9 oder 10 das Verfahren nach Anspruch 1 ausführt.

12. Computerlesbarer Datenträger, **dadurch gekennzeichnet, dass** auf dem Datenträger ein Computerprogramm nach Anspruch 11 gespeichert ist.

## Claims

1. Method for monitoring a data transmission between a first and second component (2, 3), wherein, through a change of a position and/or an orientation of the first component (2) with regard to the second component (2), one or more transmit facility (4 - 7) or transmit facilities (4 - 7) attached at fixed positions to the first component (2) are each moved with regard to at least two receive facilities (8 - 12) attached at a fixed position to the second component (3), **characterised in that**, on fulfilment of an error condition (21 - 25) for the respective receive facility (8 - 12), a respective error is registered and/or the operation of an apparatus (1) comprising the first and second component (2, 3) is modified, wherein the fulfilment of the error condition (21 - 25) for the respective receive facility (8 - 12) depends on both location information (20) determined, relating to the position and/or orientation of the first component (2) with regard to the second component (3) and also on a measure (26) for a receive quality of the signals and/or data packets (36 - 38) received from the transmit facility (4 - 7) or from at least one of the transmit facilities (4 - 7) .

2. Method according to claim 1, **characterised in that** a sequence (35) of a number of consecutive data packets (36 - 38) is sent via the respective transmit facility (4 - 7), wherein the respective data packet (36 - 38) comprises sequence information (39), which describes the position of the respective data packet (36 - 38) in the sequence (35), wherein after receipt of a number of data packets (36 - 38) by at least one of the receive facilities (8 - 12) in each case, a packet loss condition (54 - 57) is evaluated, the fulfilment of which depends on the sequence information (39) of the received data packets (36 - 38) and indicates that at least one of the data packets (36 - 38) of the sequence (35) was not received by any of the receive facilities (8 - 12), wherein on the one hand the error condition (21 - 25) is only able to be fulfilled on fulfilment of the packet loss condition (54 - 57) and/or wherein on the other hand, on fulfilment of the packet loss condition (54 - 57) a packet loss is registered and/or the operation of the apparatus (1) comprising the first and second component (2, 3) is modified.

3. Method according to claim 2, **characterised in that** the data packets (36 - 38) additionally comprise sender information (53), which describes from which of the transmit facilities (4 - 7) the respective data packet (36 - 38) was sent, wherein the packet loss condition (54 - 57) is evaluated separately in each case for a number of groups of data packets (36 - 38) of which the sender information (53) describes the same transmit facility (4 - 7) .

4. Method according to claim 2 or 3, **characterised in that** data packets (36 - 38) received from a respective transmit facility (4 - 7) or at least part information (48) of the data packets (36 - 38) relating to the sequence information (53) of the received data packets (36 - 38) are stored in a buffer (40),
wherein in each case, on or after fulfilment of a first test condition (47), of which the fulfilment depends on a number and/or a total size of the data packets (36 - 38) or part information (48) stored in a part buffer (49) of the buffer (40),
or on or after fulfilment of a second test condition (51), of which the fulfilment on the one hand depends on a number and/or a total size of the data packets (36 - 38) or part information (48) stored after fulfilment of the first test condition (47) in a further part buffer (50) of the buffer (40) and/or on the other hand depends on a time elapsed since fulfilment of the first test condition (47), for testing the packet loss condition (54 - 57), the data packets (36 - 38) or part information (48) stored in the first part buffer (49) or the data packets (36 - 38) or part information (48) stored in the first and second part buffer (49, 50) are evaluated.

5. Method according to one of the preceding claims,
**characterised in that** the first component (2) is supported rotatably on the second component (3) or vice versa, wherein the angle of rotation (41) between the first and second component (2, 3) or information correlated with this angle of rotation (41) is used as location information (20).

6. Method according to claim 5, **characterised in that** the error condition (21 - 25) is exclusively able to be fulfilled when the angle of rotation (41) lies in a predetermined range of angles of rotation (42 - 45) or in one of a number of predetermined ranges of angles of rotation (42 - 45).

7. Method according to one of the preceding claims, **characterised in that** the error condition (21 - 25) is only able to be fulfilled when a subcondition (34) evaluating the location information (20) is fulfilled.

8. Method according to one of the preceding claims, **characterised in that** the number of the receive facilities (8 - 12) used is greater by precisely one or by at least two than the number of the transmit facilities (4 - 7) used.

9. Apparatus with a first and second component (2, 3),
wherein through a change of a position and/or an orientation of the first component (2) with regard to the second component (3) one or more transmit facility (4 - 7) or transmit facilities (4 - 7) attached at a fixed position to the first component (2) are each able to be moved with regard to at least two receive facilities (8 - 12) attached at a fixed position to the second component (3), **characterised in that** the apparatus comprises means (46) that are suitable for carrying out the method according to one of the preceding claims.

10. Apparatus according to claim 9, **characterised in that** the apparatus (1) is used for medical imaging and/or that the first component (2) bears at least one imaging sensor (14) .

11. Computer program comprising commands that cause the apparatus (1) of claim 9 or 10 to carry out the method according to claim 1.

12. Computer-readable data medium, **characterised in that** a computer program according to claim 11 is stored on the data medium.

## Revendications

1. Procédé de contrôle d'une transmission de données entre un premier et un deuxième composants (2, 3), dans lequel en modifiant une position et/ou une orientation du premier composant (2) par rapport au deuxième composant (2), on déplace un dispositif (4 - 7) d'émission ou plusieurs dispositifs (4 - 7) d'émission montés fixes en position sur le premier composant (2) respectivement par rapport à au moins deux dispositifs (8 - 12) de réception montés fixes en position sur le deuxième composant (3),
**caractérisé en ce que**, si une condition (21 - 25) de défaut est satisfaite pour le dispositif (8 - 12) de réception respectif, on enregistre un défaut respectif et/ou on modifie le fonctionnement d'une installation (1) comprenant le premier et le deuxième composants (2, 3), dans lequel la satisfaction de la condition (21 - 25) de défaut du dispositif (8 - 12) de réception respectif dépend tant d'une information (20) de position déterminée, concernant la position et/ou l'orientation du premier composant (2) par rapport au deuxième composant (3), qu'également d'une valeur (26) d'une qualité de réception des signaux et/ou des paquets (36 - 38) de données reçus par le dispositif (4 - 7) d'émission ou par au moins l'un des dispositifs (4 - 7) d'émission.

2. Procédé suivant la revendication 1, **caractérisé en ce que** l'on envoie, par le dispositif (4 - 7) d'émission respectif, une séquence (35) de plusieurs paquets (36 - 38) de données successifs, dans lequel le paquet (36 - 38) respectif comprend une information (39) de séquence, qui décrit la position du paquet (36 - 38) de données respectif dans la séquence (35), dans lequel, après réception de plusieurs paquets (36 - 38) de données, par respectivement au moins l'un des dispositifs (8 - 12) de réception, on évalue une condition (54 - 57) de perte de paquets, dont la satisfaction dépend des informations (39) de séquence des paquets (36 - 38) de données reçus et est un indice qu'au moins l'un des paquets (36 - 38) de données de la séquence (35) n'a été reçu par aucun des dispositifs (8 - 12) de réception, dans lequel d'une part la condition (21 - 25) de défaut ne peut être satisfaite que si la condition (54 - 57) de perte de paquets est satisfaite et/ou dans lequel d'autre part, si la condition (54 - 57) de perte de paquets est satisfaite, on enregistre une perte de paquets et/ou on modifie le fonctionnement de l'installation (1) comprenant le premier et le deuxième composants (2, 3).

3. Procédé suivant la revendication 2, **caractérisé en ce que** les paquets (36 - 38) de données comprennent en outre une information (53) d'émission, qui décrit celui des dispositifs (4 - 7) d'émission dont le paquet (36 - 38) de données respectif a été émis, dans lequel on évalue la condition (54 - 57) de perte de paquets respectivement séparément pour plusieurs groupes de paquets (36 - 38) de données, dont les informations (53) d'émetteur décrivent le même dispositif (4 - 7) d'émission.

4. Procédé suivant la revendication 2 ou 3, **caractérisé en ce que** l'on met dans une mémoire (40) tampon des paquets (36 - 38) de données reçus d'un dispositif (4 - 7) d'émission respectif ou du moins des informations (48) partielles, concernant l'information (53) de séquence des paquets (36 - 38) de données reçus, des paquets (36 - 38) de données, dans lequel, respectivement, à la satisfaction ou après la satisfaction d'une première condition (47) de contrôle, dont la satisfaction dépend d'un nombre et/ou d'une grandeur d'ensemble des paquets (36 - 38) de données mis dans un tampon (49) partiel du tampon (40) ou d'informations (48) partielles,
ou, à la satisfaction ou après la satisfaction d'une deuxième condition (51) de contrôle, dont la satisfaction dépend d'une part d'un nombre et/ou d'une grandeur d'ensemble des paquets (36 - 38) de données mis, après la satisfaction de la première condition (47) de contrôle, dans un autre tampon (50) partiel du tampon (40) ou d'informations (48) partielles et/ou d'autre part d'un temps écoulé depuis la satisfaction de la première condition (47) de contrôle,
pour le contrôle de la condition (54 - 57) de perte de paquets, on évalue les paquets (36 - 38) de données mis dans le premier tampon (49) partiel ou des informations (48) partielles ou les paquets (36 - 38) de données mis dans le premier et le deuxième tampons (49, 50) partiels ou des informations (48) partielles.

5. Procédé suivant l'une des revendications précédentes, **caractérisé en ce que** le premier composant (2) est monté tournant sur le deuxième composant (3) ou inversement, dans lequel on utilise, comme information (20) de position, l'angle (41) de rotation entre le premier et le deuxième composants (2, 3) ou une information corrélée à cet angle (41) de rotation.

6. Procédé suivant la revendication 5, **caractérisé en ce que** la condition (21 - 25) de défaut peut être satisfaite exclusivement lorsque l'angle (41) de rotation se trouve dans une plage (42 - 45) d'angles de rotation donnée à l'avance ou dans une plage parmi plusieurs plages (42 - 45) d'angles de rotation données à l'avance.

7. Procédé suivant l'une des revendications précédentes, **caractérisé en ce que** la condition (21 - 25) de défaut ne peut être satisfaite que lorsqu'une condition (34) partielle évaluant l'information (20) de position est satisfaite.

8. Procédé suivant l'une des revendications précédentes, **caractérisé en ce que** le nombre des dispositifs (8 - 12) de réception utilisés est plus grand exactement de un ou d'au moins deux que le nombre des dispositifs (4 - 7) d'émission utilisés.

9. Installation comprenant un premier et un deuxième composants (2, 3), dans laquelle, en modifiant une position et/ou une orientation du premier composant (2) par rapport au deuxième composant (3), un ou plusieurs dispositifs (4 - 7) d'émission ou dispositifs (4 - 7) d'émission montés fixes en position sur le premier composant (2) peuvent être déplacés respectivement par rapport au moins deux dispositifs (8 - 12) de réception montés fixes en position sur le deuxième composant (3), **caractérisée en ce que** l'installation comprend des moyens (46), qui sont propres à effectuer le procédé suivant l'une des revendications précédentes.

10. Installation suivant la revendication 9, **caractérisée en ce que** l'installation (1) sert à l'imagerie médicale et/ou **en ce que** le premier composant (2) porte au moins un capteur (14) donnant une image.

11. Programme d'ordinateur comprenant des instructions, qui font que l'installation (1) de la revendication 9 ou 10 effectue le procédé suivant la revendication 1.

12. Support de données, déchiffrables par ordinateur, **caractérisé en ce qu'**un programme d'ordinateur suivant la revendication 11 est mis en mémoire sur le support de données.
